# EUROPEAN PATENT APPLICATION

(11) **EP 4 644 902 A1**
(43) Date of publication of application: **05.11.2025**
(21) Application number: 24173950.7
(22) Date of filing: 02.05.2024
(51) Int. Cl.: G01N 33/68, C07K 16/28

(54) **ANTI NAV1.5 CHANNEL AUTOANTIBODIES AS BIOMARKERS FOR PREDICTING THE RISK OF CARDIAC ARRHYTHMIA AND SUDDEN CARDIAC DEATH IN CANCER PATIENTS**

(71) Applicant: Cardiomix S.r.l., 20121 Milano (IT)
(72) Inventor: Anastasia, Luigi, 20121 Milan (IT); Tarantino, Adriana, 20121 Milan (IT); Ciconte, Giuseppe, 20121 Milan (IT); Pappone, Carlo, 20121 Milan (IT)
(74) Representative: Bird & Bird Società tra Avvocati S.r.l.

(57) **Abstract**

The present invention relates to autoantibodies targeting the human NaV1.5 sodium channel and related isoforms as biomarkers for risk prediction of cardiac arrhythmias and sudden cardiac death in cancer patients, related methods and diagnostic kit for their detection.

## Description

The present invention relates to autoantibodies targeting the human NaV1.5 sodium channel and related isoforms as biomarkers for risk prediction of cardiac arrhythmias and sudden cardiac death in cancer patients, related methods and diagnostic kit for their detection.

Sudden cardiac death (SCD) in cancer patients represents a significant clinical challenge, often occurring unexpectedly and leading to significant mortality, ranging from 6 to 20% in the first six months of treatment ([1], [2]).

The increased prevalence of cardiovascular deaths and unexpected sudden death among patients with advanced cancer is a complex phenomenon that cannot be solely attributed to the direct cardiotoxic effects of treatments like chemotherapy ([1]-[4]). This complexity underscores the urgent need for identifying biomarkers capable of predicting the risk of SCD, especially during periods when the heart is under severe stress due to therapeutic interventions [4].

The voltage-gated sodium NaV1.5 channel, and specifically its α subunits, have attracted considerable attention due to their roles in cardiac arrhythmias and autoimmune responses ([5]-[8]).

The present inventors have recently discovered the presence of circulating autoantibodies targeting the NaV1.5 channel in a cohort of 100 patients with Brugada Syndrome (BrS) (unpublished data, pending European patent applications No. EP23187367.0 and No. EP24161814.9, herein included as reference). Notably, the infusion of plasma from BrS patients containing these autoantibodies into mice has resulted in the induction of severe arrhythmic events and the replication of distinctive electrocardiogram (ECG) patterns akin to those observed in Brugada patients. Moreover, it has been additionally observed that autoantibodies isolated from plasma of Brugada syndrome patients decrease inward sodium current (of approximately 40%) in HEK293 cells overexpressing NaV1.5 channel. All the above findings not only support the diagnostic significance of these autoantibodies but also implicates them as active contributors to the pathogenesis of the syndrome. Beyond their traditional role in excitable cells, voltage-gated sodium channels (VGSCs), including NaV1.5, are expressed in a range of "non-excitable" cells, such as immune cells and several types of metastatic cancer cells [9].

These channels play a critical role in cell migration and invasion, emphasizing their diagnostic and therapeutic importance. Indeed, the α subunits have been identified in different solid and liquid cancers of epithelial origins *in-vitro* and *in-vivo,* such as breast cancer, cervical cancer, colon cancer, melanoma, mesothelioma, neuroblastoma, non-small cell lung cancer, ovarian cancer, prostate cancer, small-cell lung cancer [10], gliomas [11], lymphoma [12] and leukemia cells [13].

In this context, it seems that certain sodium channel isoforms are selectively expressed types of cancers [14], with varying patterns of expression across different α subunits [15].

The oncogenic potential attributed to sodium channel activity in tumors is notably linked to the splice variant of mRNA encoding the neonatal form of NaV1.5 (nNaV1.5). This splicing event takes place in exon 6, responsible for encoding the domain I segment 3 (DI: S3) region, whereas the 3' adult variant emerges in subsequent stages of postnatal development ([14], [16], [17]). Along this line, the 7 amino acids in the extracellular loop in VSD1 (D1:S3-S4) of nNaV1.5 is an highly antigenic sequence (18, 19), which has been associated with the presence of antibodies against nNaV1.5 channel in patients with breast cancer compared to those who received the treatments in which the expression in tumor tissue of the neonatal form of the Nav1.5 was dramatically reduced [20].

Elevated occurrences of cardiovascular deaths (CVDs) ([1],[2]) or unexpected sudden death (USD) [1] have been observed among patients with advanced cancer, often associated with non-cancer related factors. However, the increased prevalence of these events, for example, in breast cancer patients compared to the general population ([2],[21]), cannot be solely explained by cardiotoxic effects of treatments like chemotherapy [22].

The authors of present invention have now surprisingly found that autoantibodies that bind the adult isoform of NaV1.5 in the heart are valuable biomarkers for the detection of the risk of sudden cardiac death in cancer patients.

Having in mind the crucial role that autoantibodies targeting the NaV1.5 channel present in the blood of Brugada patients play, being the cause of the typical ECG phenotype leading to cardiac arrhythmias, the authors of the present invention investigated whether a similar pathophysiological mechanism is at play in cancer patients. Therefore, it was investigated whether the overexpression of NaV1.5 channel in cancer cells leads to the formation of autoantibodies against it, which could ultimately cause sudden cardiac death in cancer patients as well.

To date, a single study previously found autoantibodies against the neonatal isoform of NaV1.5 channel in breast cancer patients who were undergoing chemotherapy [20]. However, there is no report of autoantibodies directed against the adult cardiac isoform of NaV1.5 in cancer patients or evidence that such autoantibodies could cause heart issues potentially leading to sudden cardiac death.

The significance of autoantibodies against the NaV1.5 channel, as well as against all other ion channels like those for calcium and potassium, had been unclear ([7], [23]). Prior to the groundbreaking discovery of anti-NaV1.5 autoantibodies in Brugada syndrome recently carried out by the present inventors, only one study had made a connection between arrhythmias and the presence of anti-NaV1.5 autoantibodies [5], but not in relation with cancer patients. This study simply demonstrated that rats immunized with one of the external loops of NaV1.5 developed heart conduction problems typical of idiopathic AV blocks [5].

Therefore, it is an object of the present invention antibodies for use as biomarkers for the prediction of risk of cardiac arrhythmia or sudden cardiac death (SCD) in patients suffering from cancers overexpressing sodium channels, said antibodies being directed against NaV 1.5 channel (SEQ ID NO:1) and/or related isoforms. Thus, the major objective of the present invention is to use antibodies as biomarkers to stratify the risk of sudden cardiac death (SCD) in individuals, in particular against the NaV1.5 channel (SEQ ID NO:1) and its related isoforms. In fact, the autoantibodies targeting the NaV1.5 channel (SEQ ID NO:1) may be cross-reactive with other NaV1.5 channel isoforms. Due to the high sequence similarity between the canonical NaV1.5 channel and its isoforms, including the embryonic variant nNaV1.5, autoantibodies originally raised against these alternative isoforms may also interact with the cardiac NaV1.5 channel.

In fact, it has been observed that autoantibodies targeting NaV1.5 channel (SEQ ID NO:1) may be cross reactive with other NaV1.5 channel isoforms. Due to the high sequence similarity between the canonical NaV1.5 channel and its isoforms, including the embryonal variant nNaV1.5, autoantibodies initially generated against these alternative isoforms may also interact with the cardiac NaV1.5 channel. Such molecular mimicry can contribute to the mechanism leading to sudden cardiac death in cancer patients by affecting the functional integrity of the cardiac sodium channel, potentially exacerbating the risk of cardiac arrhythmias.

Therefore, according to the present invention the expression "NaV1.5 channel and related isoforms," encompasses any and all variants of the NaV1.5 channel, including but not limited to the embryonal isoform, which share significant sequence homology with the canonical NaV1.5 channel (SEQ ID NO: 1). With the expression "significant sequence homology" it is intended a sequence homology greater than 85%, preferably greater than 90%, even more preferably greater than 95%, 96%, 97%, 98% or 99%.

In a preferred embodiment the antibodies for use as biomarkers for the prediction of risk of cardiac arrhythmia or sudden cardiac death in patients suffering from cancer overexpressing sodium channels are directed against a binding site of the extracellular loops of NaV 1.5 channel (SEQ ID NO:1) and/or related isoforms. However, it cannot be excluded that in the presence of a misfolded protein channel in cancer patients overexpressing sodium channels the autoantibodies may be directed also against a binding site exposed in the inner channel protein.

Preferably, the antibodies for use as biomarkers for the prediction of risk of cardiac arrhythmia or sudden cardiac death in patients suffering from cancer overexpressing sodium channels are directed against a binding site of the extracellular loops of NaV 1.5 channel (SEQ ID NO:1) and/or related isoforms, wherein said binding site may be selected from the group comprising the following sequences:
i) VFALIGLQLFMGNLRHKCVRNFTALNGTNGSVEADGLVWESLDLYLS DPENYLLKNGTSDVLLCGNSSDAGTCPEGYRCLKAGENPDHGYTSFDSFA WAFLALFRL (SEQ ID NO:2)
ii) FGKNYSELRDSDSGLLPRWHMMDFFHAFLIIFRILCG (SEQ ID NO:3)
iii) SIMGVNLFAGKFGRCINQTEGDLPLNYTIVNNKSQCESLNLTGELYW TKVKVNFDNVGAGYLALLQ (SEQ ID NO: 4)
iv) VILSIVGTVLSDIIQKYFFSPTLFRVIRLARIGRIL (SEQ ID NO:5)
v) IYSIFGMANFAYVKWEAGIDDMFNFQTFANSMLCLFQI (SEQ ID NO:6)
vi) AGWDGLLSPILNTGPPYCDPTLPNSNGSRGDCGSPAVGILFFTT (SEQ ID NO:7)
or fragments thereof.

According to a preferred embodiment of the present invention said cancer overexpressing sodium channels is selected from the group consisting of breast cancer, colon cancer, prostate cancer, cervical cancer, melanoma, mesothelioma, neuroblastoma, non-small cell lung cancer, ovarian cancer, thyroid cancer, small-cell lung cancer and lymphoma.

The invention further relates to an in vitro method for the prediction of risk of cardiac arrhythmia or sudden cardiac death in a patient suffering from cancer overexpressing sodium channels, comprising the following steps:
a) contacting a biological sample of the patient suffering from cancer with one or more antigens that specifically binds to the antibodies directed against NaV 1.5 channel (SEQ ID NO:1) and/or related isoforms as above disclosed; and
b) detecting the binding of the antigen to the antibodies in said biological sample.

According to a preferred embodiment of the present invention said cancer overexpressing sodium channels is selected from the group consisting of breast cancer, colon cancer, prostate cancer, cervical cancer, melanoma, mesothelioma, neuroblastoma, non-small cell lung cancer, ovarian cancer, thyroid cancer, small-cell lung cancer and lymphoma.

In a preferred embodiment of the invention, said antibodies are directed against extracellular loops of the NaV 1.5 channel of (SEQ ID NO:1) and/or related isoforms.

More preferably said antigens are antigenic fragments of at least 7 amino acids from the binding sites of extracellular loops of NaV1.5 channel. In a preferred embodiment said binding sites of extracellular loops of NaV1.5 channel are selected between the sequences SEQ ID NO:2-SEQ ID NO:7.

According to a preferred embodiment of the in vitro method of detection of antibodies against NaV1.5 channel and related isoforms of the invention, said antigens are labelled or attached to a solid support.

In a further preferred embodiment of the in vitro method of the invention the biological sample is selected from the group consisting of plasma, PBMCs, whole blood, serum and peripheral blood, or a combination thereof. Preferably, the biological sample is plasma and/or PBMCs.

In a preferred embodiment of the in vitro method of detection of antibodies against NaV1.5 channel and related isoforms of the invention, the detection of the binding of the antigens to the antibodies is performed by ELISA or Western blotting.

The present invention is further directed to a diagnostic kit comprising one or more antigens that specifically binds to the antibodies against NaV 1.5 channel (SEQ ID NO:1) and/or related isoforms as above disclosed, for the prediction of the risk of cardiac arrythmia or sudden cardiac death in a patient suffering from cancer overexpressing sodium channels. Preferably, said one or more antigens specifically bind to the antibodies directed against extracellular loops of NaV1.5 channel of (SEQ ID NO:1). Even more preferably said antigens comprises one or more antigenic fragments belonging to the binding site of the extracellular loops of the NaV1.5 protein, said binding site of the extracellular loops being selected from the group of sequences SEQ ID NO:2-SEQ ID NO:7.

In a preferred embodiment of the diagnostic kit of the invention, said antigens are labelled or attached to a solid support. Preferably, said antibodies are labelled with fluorescents.

Said solid support is preferably a multi-well plate. Preferably, said kit is an ELISA kit.

Moreover, the invention is directed to the use of one or more antibodies against NaV 1.5 channel (SEQ ID NO:1) and/or related isoforms as above disclosed for the prediction of risk of cardiac arrythmia or sudden cardiac death in a patient suffering from cancer overexpressing sodium channels, wherein the detection of the presence of such antibodies in a biological sample of said patient indicates an elevated risk, wherein said biological sample is selected from the group consisting of plasma, PBMCs, whole blood, serum and peripheral blood, or a combination thereof.

According to a preferred embodiment of the invention, said detection step is carried out by using one or more antigens that specifically binds to the autoantibodies against NaV 1.5 channel (SEQ ID NO:1) and/or related isoforms. In a further preferred embodiment of the invention said one or more antigens are selected from the antigenic fragments belonging to a binding site of the extracellular loop of NaV 1.5 channel consisting of the sequences SEQ ID NO:2-SEQ ID NO:7.

The present invention will now be described, for non-limiting illustrative purposes, according to a preferred embodiment thereof, with reference to the attached figures, wherein:

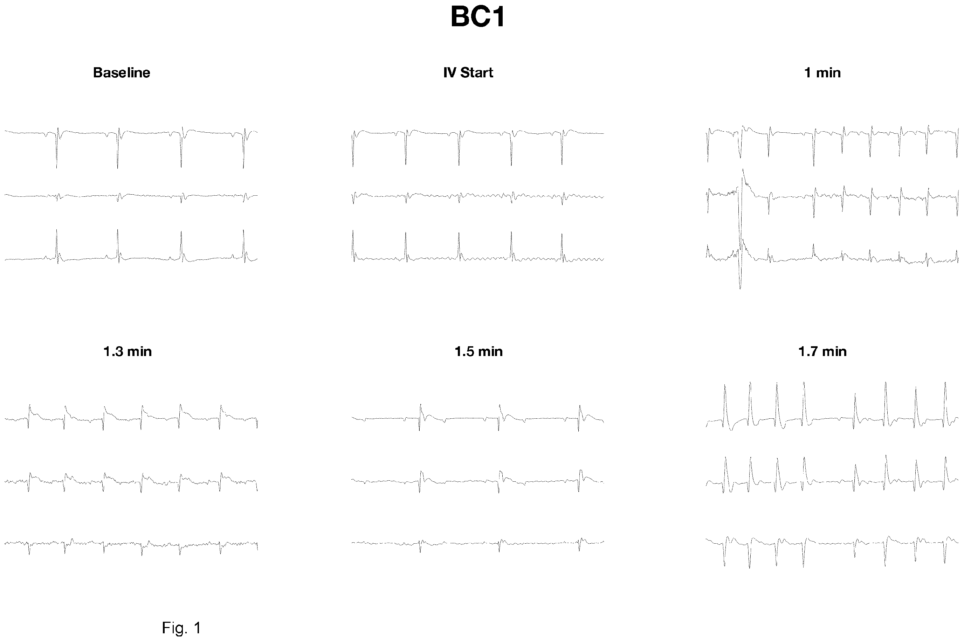

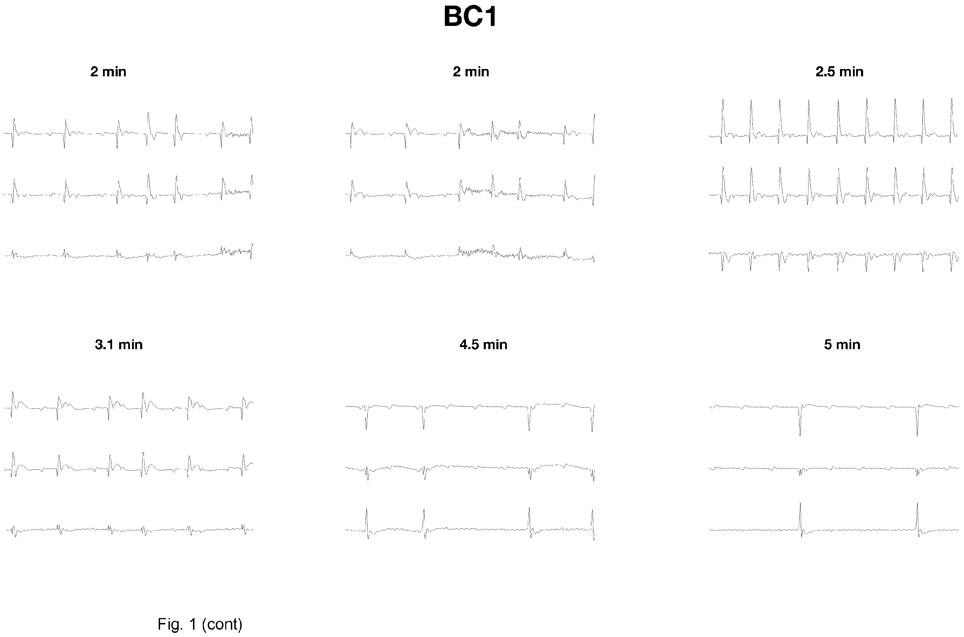

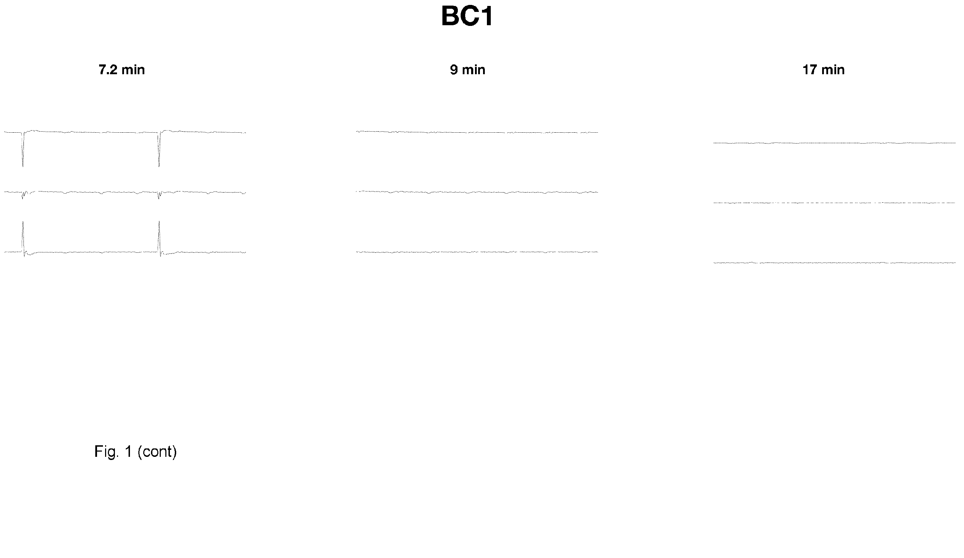

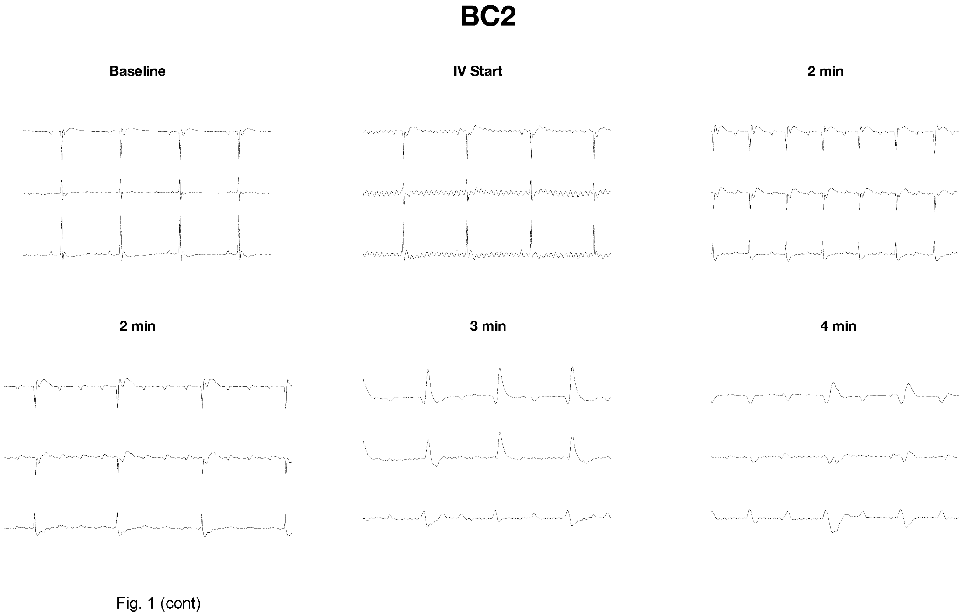

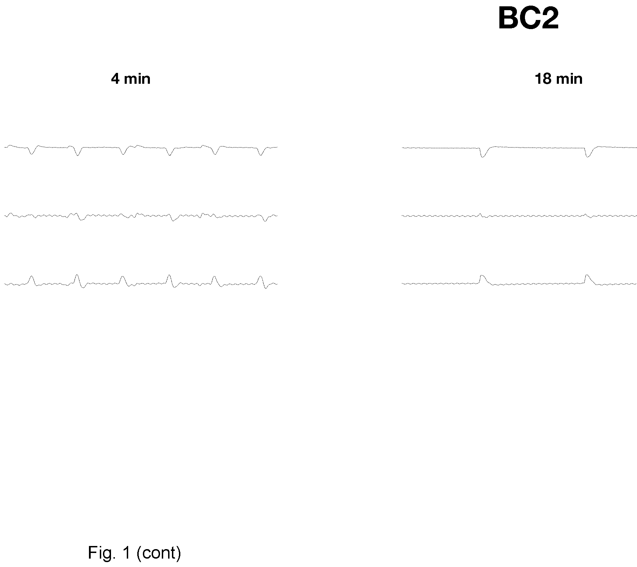

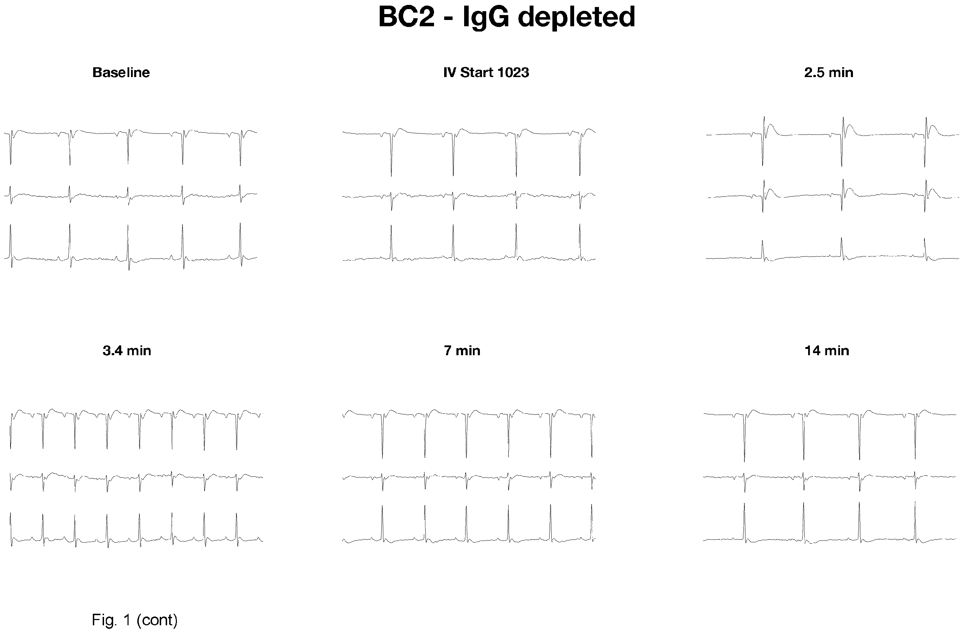

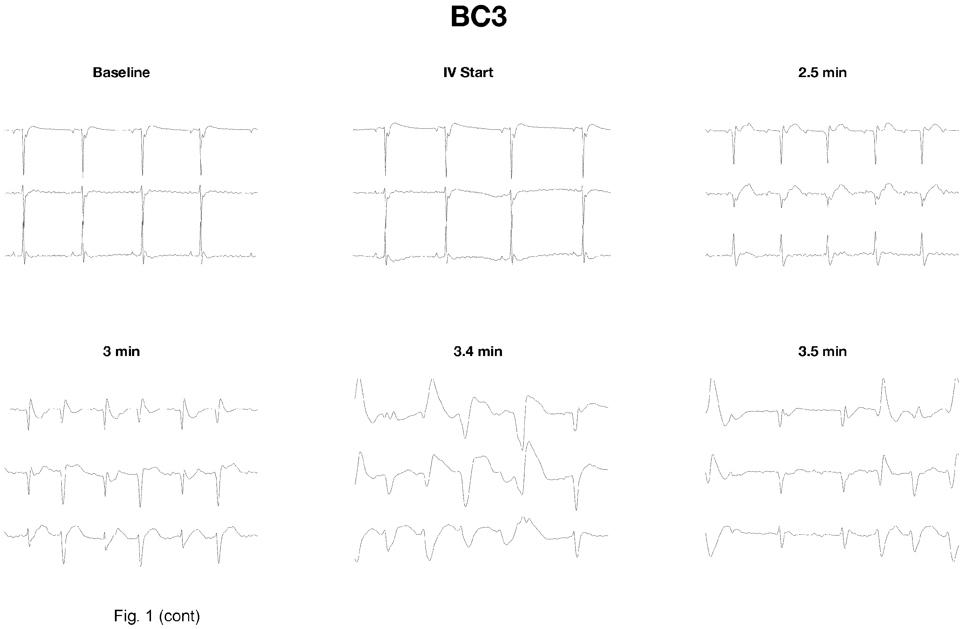

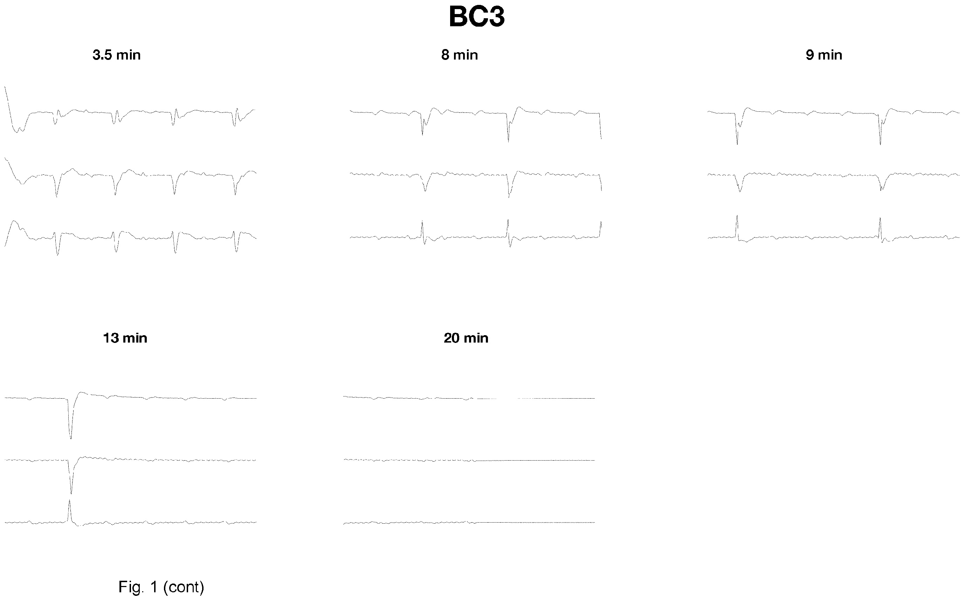

- Figure 1 shows a series of electrocardiogram (ECG) traces demonstrating the effects of injecting plasma containing autoantibodies from breast cancer (BC) patients into mice. Panel A (BC1): ECG traces before and after injection with autoantibodies from the first breast cancer patient (BC1). Panel B (BC2): Similar ECG traces for the second breast cancer patient (BC2), from baseline and at successive times (2, 3, 4, and 18 minutes) post-injection, depict progression to fatal arrhythmias. Panel C (BC2 - IgG depleted): ECG traces following the injection of IgG-depleted plasma from the same BC2 patient.

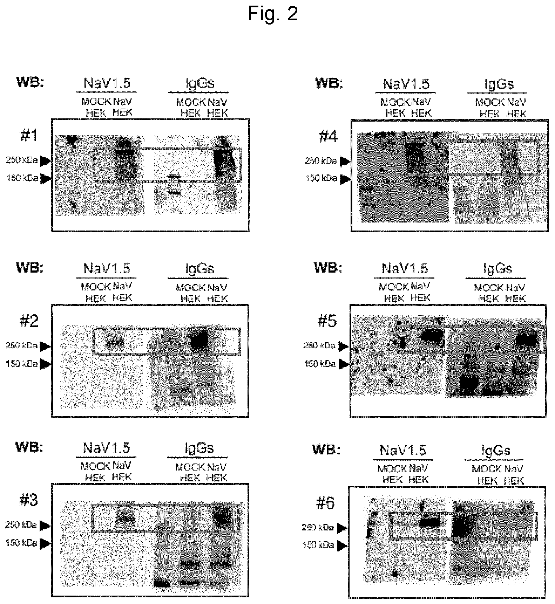

- Figure 2 shows Western blot analysis illustrating the detection of autoantibodies targeting NaV1.5 in plasma from breast cancer patients.

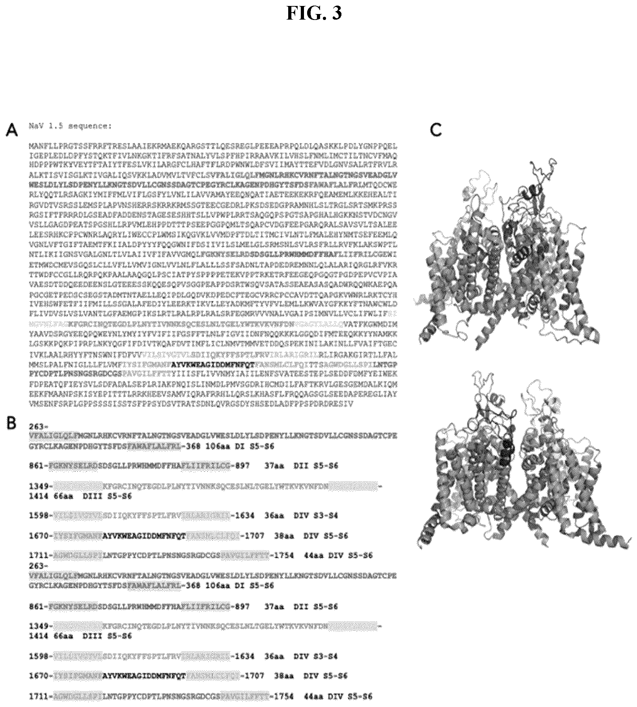

- Figure 3 shows the identification of putative binding sites of autoantibodies on NaV1.5 protein. Panel A) Amino acid sequence ofNaV1.5 channel (SEQ ID NO:1); Panel B) Identification of six potential regions that serve as binding sites for autoantibodies. The 10 amino acids critical for binding on the solid support are highlighted in yellow; Panel C) PyMol structure showing the core of the NaV1.5 channel and the extracellular loop.

The following examples are merely illustrative and should not be considered limiting the scope of the present invention.

### EXAMPLE 1: ECG Alterations in Mice Following Injection of Plasmafrom Cancer Patients with Anti-NaV1.5 Antibodies

### METHODS

### Plasma Collection

Healthy wild-type mice were injected with plasma from patients with breast cancer (BC) and colon cancer (data not shown).

Blood samples from 10 BC patients (25 ml) were centrifuged at 1000xg for 15 minutes to separate plasma. A second centrifugation at 2000xg for 15 minutes further clarified the plasma, which was then aliquoted and stored at -20 °C.

### Generation of Stable Cell Line and Transient Transfections

For each of the three breast cancer patient samples (#1, #2, #3), HEK293 cells were transiently transfected with either a control vector (Mock HEK) or a vector expressing NaV1.5 (NaV HEK).

HEK293A cells were cultured in Dulbecco's Modified Eagle Medium (DMEM, Life Technologies) supplemented with 10% fetal bovine serum (FBS, Sigma), 2 mM glutamine (Merck), and 1X penicillin/streptomycin (Euroclone), at 37°C in a humidified atmosphere of 5% CO2 and 95% air.

The NaV1.5 plasmid was synthesized, encoding the full-length human NaV1.5 cDNA, and cloned into the pcDNA 3.1(+) vector. Transfections were performed using j etPRIME (Euroclone), following the manufacturer's protocol, with cells later selected using G418.

### NaV1.5 Immunoblotting

HEK293A cells overexpressing NaV1.5 were lysed using RIPA buffer containing a cocktail of protease and phosphatase inhibitors. The supernatant was then centrifuged at 15,000 rpm for 10 minutes at 4 °C and collected. The protein concentrations were determined using the BCA assay (Pierce). Proteins were denatured and reduced with Laemmli buffer containing β-mercaptoethanol (Bio-Rad) and loaded onto a 10% SDS-PAGE gel (Protean Tgx Stain-Free, Bio-Rad) for electrophoresis and then transferred to nitrocellulose membranes.

Initially, the membranes were blocked and probed overnight at 4°C with a commercial anti-NaV1.5 antibody (dilution 1:2000, Cell Signaling, clone D9J7S). They were then washed and incubated with a secondary anti-rabbit IRDye 800 CW antibody (dilution 1:2000, LI-COR Biosciences) to confirm overexpression of NaV1.5 in the transfected cells, as evidenced by a prominent band at approximately 250 kDa in the NaV HEK lanes and no corresponding band in the mock HEK lanes. Subsequently, the membranes were incubated with plasma from breast cancer patients and detected with an anti-human IgG-HRP antibody (dilution 1:2000, Bio-Rad) to identify autoantibodies against NaV1.5. The presence of bands at 250 kDa in the NaV HEK lanes incubated with patient plasma, which match the position of the NaV1.5 band identified with the commercial antibody, indicates binding of the patient-derived autoantibodies to the NaV1.5 channel. Bands were visualized using the ECL Advance Kit (GE Healthcare) and imaged using the ChemiDoc MP System (Bio-Rad).

### Animals and Electrocardiography

The procedure involving mice was performed according to the animal protocol guidelines described by the Institutional Animal Care and Use Committee (IACUC) authorization no. 425/2022/PR at San Raffaele Scientific Institute (Milan, Italy). Mice C57BL-6 at 50 weeks were maintained ad libitum access to water and standard chow food at room temperature with a 12-h light/dark schedule. They were anesthetized by intraperitoneal injection of medetomidine, 0.5 mg/kg (Orion Pharma S.r.l.) and ketamine, 100 mg/kg (Merial), both diluted in saline solution. Body weight was determined prior to each investigation, the body temperature was constantly monitored and kept at 37 ± 0.5°C by a homeothermic blanket system with a rectal thermometer probe (Harvard Apparatus, Holliston, Massachusetts, USA). 200 ml of plasma from BrS (n=4) and CTR (n=3) were pre-heated at 56 °C for 20 min before the intravenous injection in anesthetized mice. Briefly, ECG was performed continuously from 10 minutes after induction of anesthesia and 30 minutes after plasma injection using three subcutaneous needle electrodes (stainless steel, 27-gauge, 12 mm length; SEI EMG s.r.l., Cittadella, Italy): two needles were inserted in the forelimbs and one in the left hindlimb, and another needle electrode was placed in the right hindlimb as a ground. Needle electrodes were connected via flexible cables to an amplifier (Micromed, Mogliano Veneto, Italy), then the ECG signal was recorded using System-Plus software (Micromed, Mogliano Veneto, Italy) and sampled at 256 Hz (16 bits) with band-pass filters between 1 and 70 Hz. All animal experiments were performed without prior knowledge of the origin of the plasma samples to ensure the integrity of the results.

### RESULTS

The mice injected with plasma from breast cancer patients showed ECG trace alterations comparable to those of patients with Brugada syndrome. In fact, just a few minutes after injection, the mice showed the typical type 1 ST-segment elevation observed in humans with BrS, followed by a severe arrhythmic phenotype characterized by life-threatening ventricular arrhythmias and instances of complete AV block, ultimately leading to the death of the mice within 20 minutes.

In addition, the mice exhibited ECG abnormalities such as ventricular arrhythmias and complete AV blocks, which eventually led to the death of the mice (Figure 1). Panels A-D of Figure 1 presents data from a different scenario, showcasing the progression towards severe arrhythmias and the eventual fate of the mice:
- Panel A (BC1) shows ECG traces before and after injection with autoantibodies from the first breast cancer patient (BC1). Traces captured at various intervals (1.3, 1.5, 1.7, 2, 2.5, 7.2, 9, and 17 minutes) post-injection show rapid development of arrhythmias. The mouse succumbs to the arrhythmic events shortly after the last recorded time point.
- Panel B (BC2) shows similar ECG traces for the second breast cancer patient (BC2), from baseline and at successive times (2, 3, 4, and 18 minutes) post-injection, depict progression to fatal arrhythmias. The mouse dies due to these cardiac disturbances.
- Panel C (BC2 - IgG depleted) shows ECG traces following the injection of IgG-depleted plasma from the same BC2 patient. Time points (2.5, 3.4, 7, and 14 minutes) show that the ECG abnormalities are less severe, and notably, the ECG quickly normalizes, returning to baseline conditions. This mouse survives, highlighting the pivotal role of autoantibodies in mediating the observed cardiac effects. Remarkably, removal of IgGs from patient plasma using protein G-coated beads effectively abolished these effects and underscored that the observed effects on the heart were indeed mediated by autoantibodies.
- Panel D (BC3): ECG traces from the third breast cancer patient (BC3), showing the sequence from baseline through various intervals (2.5, 3, 3.4, 3.5, 8, 9, 13, and 20 minutes) post-autoantibody injection. The progression to severe arrhythmias is evident, leading to the mouse's death.

This discovery not only underscores the potential of circulating autoantibodies against the NaV1.5 channel as a causative factor for sudden cardiac death in cancer patients, but also highlights the conserved nature of NaV1.5 isoforms, suggesting that autoantibodies can target the cardiac isoform despite being directed against specific isoforms due to their high sequence similarity.

This important finding was confirmed by Western blot assays, which showed that the autoantibodies bind to the wild-type isoform of the cardiac sodium NaV1.5 channel under denaturing conditions (Figure 2).

Figure 2 shows Western blot analysis illustrating the specific detection of NaV1.5 protein and subsequent identification of patient-derived autoantibodies binding to NaV1.5.

Notably, all three patient samples showed evidence of autoantibody binding, with varying degrees of intensity, suggesting the presence of autoantibodies against the NaV1.5 channel in the plasma of breast cancer patients, which could have implications for cardiac arrhythmias and sudden cardiac death risk.

This invention represents a pioneering approach in the field of medical diagnostics and therapy. It targets the critical and previously under-researched intersection of cancer, autoimmunity and sudden cardiac death (SCD) through the lens of NaV1.5 channel autoantibodies. The presence of such autoantibodies that specifically bind to the adult cardiac isoform of NaV1.5 reveals a novel pathophysiologic mechanism potentially leading to sudden cardiac death in cancer patients, a population not previously associated with this specific autoimmune response.

The study not only highlights the potential of NaV1.5 channel autoantibodies as biomarkers for risk stratification of sudden cardiac death in cancer patients, but also illuminate their broader diagnostic and therapeutic implications across a spectrum of diseases characterized by altered sodium channel expression.

### ESEMPIO 2: Prediction of Putative NaV1.5 Autoantibody Binding-Sites on NaV1.5

### Channel Protein

The human NaV1.5 channel has the following amino acid sequence:










































 (SEQ ID NO:1)

Using molecular modelling, the authors identified six (DI-DVI) putative binding sites for autoantibodies on the NaV1.5 channel protein extracellular loops (in bold in SEQ ID NO:1) as represented in Figure 3:
**DI S5-S6 (263-368) 106 aa:** VFALIGLQLFMGNLRHKCVRNFTALNGTNGSVEADGLVWESLDLYLSDP 
ENYLLKNGTSDVLLCGNSSDAGTCPEGYRCLKAGENPDHGYTSFDSFAW
AFLALFRL (SEQ ID NO:2)
**DII S5-S6 (861-897) 37 aa:**
   FGKNYSELRDSDSGLLPRWHMMDFFHAFLIIFRILCG (SEQ ID NO:3)
**DIII S5-S6 (1349-1414) 66 aa** 
SIMGVNLFAGKFGRCINQTEGDLPLNYTIVNNKSQCESLNLTGELYWTKV
KVNFDNVGAGYLALLQ (SEQ ID NO: 4)
**DIV S3-S4 (1598-1634) 36 aa**
   VILSIVGTVLSDIIQKYFFSPTLFRVIRLARIGRIL (SEQ ID NO:5)
**DV S5-S6 (1670-1707) 38 aa**
   IYSIFGMANFAYVKWEAGIDDMFNFQTFANSMLCLFQI (SEQ ID NO:6)
**DVI S5-S6 (1711-1754) 44aa**
   AGWDGLLSPILNTGPPYCDPTLPNSNGSRGDCGSPAVGILFFTT (SEQ ID NO:7)

### REFERENCES

1. Taniyama T, Tokutani R, Hiramoto S. Palliat Support Care. 2022; 20(6):818-22.
2. Guan T, Zhang H, Yang J, Lin W, Wang K, Su M, et al. Front Oncol. 2020; 10: 619622.
3. Ito S, Morita T, Uneno Y, Taniyama T, Matsuda Y, Kohara H. Cancer Med. 2021; 10(14):4939-47.
4. Sherazi S, Patel A, Hsu K, Schleede S, Watts A, McNitt S, et al. Risk of Sudden Cardiac Death in Patients Undergoing Cancer Treatment. Am J Cardiol. 2024; 214:136-41.
5. Korkmaz S, Zitron E, Bangert A, Seyler C, Li S, Hegedus P. J Am Coll Cardiol. 2013; 62(4):340-9.
6. Rajaratinam H, Mokhtar NF, Asma-Abdullah N, Fuad WEM. Biomolecules. 2022;12(2).
7. Swirski FK, Nahrendorf M. Nat Rev Immunol. 2018; 18(12):733-44.
8. Li J. Curr Cardiol Rep. 2020; 23(1):3.
9. Brackenbury WJ, Djamgoz MB, Isom LL. Neuroscientist. 2008; 14(6):571-83.
10. Brackenbury WJ. Channels (Austin). 2012; 6(5):352-61.
11. Schrey M, Codina C, Kraft R, Beetz C, Kalff R, Wolfl S, Patt S. Neuroreport. 2002; 13(18):2493-8.
12. Fraser SP, Diss JK, Lloyd LJ, Pani F, Chioni AM, George AJ, Djamgoz MB. FEBS Lett. 2004; 569(1-3):191-4.
13. Yamashita N, Hamada H, Tsuruo T, Ogata E. Cancer Res. 1987;47(14):3736-41.
14. Fleishman DG, Nikiforov VA, Saulus AA. Biol Mass Spectrom. 1992; 21(2): 80-4.
15. Diss JK, Archer SN, Hirano J, Fraser SP, Djamgoz MB. Prostate. 2001; 48(3): 165-78.
16. Onkal R, Mattis JH, Fraser SP, Diss JK, Shao D, Okuse K, Djamgoz MB. Alternative splicing of Nav1.5: an electrophysiological comparison of'neonatal' and 'adult' isoforms and critical involvement of a lysine residue. J Cell Physiol. 2008;216(3):716-26.
17. Ou SW, Kameyama A, Hao LY, Horiuchi M, Minobe E, Wang WY, et al. Tetrodotoxin-resistant Na+ channels in human neuroblastoma cells are encoded by new variants of Nav1.5/SCN5A. Eur J Neurosci. 2005;22(4):793-801.
18. Murtadha AH, Azahar IIM, Sharudin NA, Che Has AT, Mokhtar NF. Influence of nNav1.5 on MHC class I expression in breast cancer. J Biosci. 2021;46.
19. Fraser SP, Onkal R, Theys M, Bosmans F, Djamgoz MBA. Neonatal Na(V) 1.5 channels: pharmacological distinctiveness of a cancer-related voltage-gated sodium channel splice variant. Br J Pharmacol. 2022;179(3):473-86.
20. Raj aratinam H, Rasudin NS, Al Astani TAD, Mokhtar NF, Yahya MM, Zain WZW, et al. Breast cancer therapy affects the expression of antineonatal Nav1.5 antibodies in the serum of patients with breast cancer. Oncol Lett. 2021;21(2): 108.
21. He J, Wang S, Liu H, Duan C, Zhang H, Wen F, Zhang C. Competing risk analysis of cardiovascular death in breast cancer: evidence from the SEER database. Transl Cancer Res. 2023;12(12):3591-603.
22. Agha A, Wang X, Wang M, Lehrer EJ, Horn SR, Rosenberg JC, et al. Long-Term Risk of Death From Heart Disease Among Breast Cancer Patients. Front Cardiovasc Med. 2022;9:784409.
23. Lazzerini PE, Laghi-Pasini F, Boutjdir M, Capecchi PL. Cardioimmunology of arrhythmias: the role of autoimmune and inflammatory cardiac channelopathies. Nat Rev Immunol. 2019;19(1):63-4.

## Claims

1. Antibodies directed against NaV 1.5 channel (SEQ ID NO:1) and/or related isoforms, for use as biomarkers for the prediction of risk of cardiac arrhythmia or sudden cardiac death in patients suffering from cancer overexpressing sodium channels.

2. Antibodies for use according to claim 1, directed against a binding site of the extracellular loops of NaV 1.5 channel (SEQ ID NO:1) and/or related isoforms.

3. Antibodies for use according to claim 2, wherein said binding site of the extracellular loops of NaV 1.5 channel and/or related isoforms is selected from the group comprising the following sequences:
i) VFALIGLQLFMGNLRHKCVRNFTALNGTNGSVEADGLVWESLDLYLS DPENYLLKNGTSDVLLCGNSSDAGTCPEGYRCLKAGENPDHGYTSFDSFA WAFLALFRL (SEQ ID NO:2)
ii) FGKNYSELRDSDSGLLPRWHMMDFFHAFLIIFRILCG (SEQ ID NO:3)
iii) SIMGVNLFAGKFGRCINQTEGDLPLNYTIVNNKSQCESLNLTGELYW TKVKVNFDNVGAGYLALLQ (SEQ ID NO: 4)
iv) VILSIVGTVLSDIIQKYFFSPTLFRVIRLARIGRIL (SEQ ID NO:5)
v) IYSIFGMANFAYVKWEAGIDDMFNFQTFANSMLCLFQI (SEQ ID NO:6)
vi) AGWDGLLSPILNTGPPYCDPTLPNSNGSRGDCGSPAVGILFFTT (SEQ ID NO:7)
or fragments thereof.

4. Antibodies for use according to anyone of claims 1-3, wherein said cancer overexpressing sodium channels is selected from the group consisting of breast cancer, colon cancer, prostate cancer, cervical cancer, melanoma, mesothelioma, neuroblastoma, non-small cell lung cancer, ovarian cancer, thyroid cancer, small-cell lung cancer and lymphoma.

5. An in vitro method for the prediction of risk of cardiac arrhythmia or sudden cardiac death in a patient suffering from cancer overexpressing sodium channels, comprising the following steps:
a) contacting a biological sample of the patient suffering from cancer with one or more antigens that specifically binds to the antibodies directed against NaV 1.5 channel (SEQ ID NO:1) and related isoforms according to anyone of claims 1-4; and
b) detecting the binding of the antigen to the antibodies in said biological sample.

6. An in vitro method according to claim 5, wherein said one or more antigens are labelled or attached to a solid support.

7. An in vitro method to anyone of claims 5-6, wherein said antigens are antigenic fragments of at least 10 amino acids from the binding sites of extracellular loops of NaV1.5 channel.

8. An in vitro method according to claim 7, wherein said binding sites of extracellular loops of NaV1.5 channel are selected between SEQ ID NO:2-SEQ ID NO:7 or fragments thereof.

9. An in vitro method according to anyone of claims 5-8, wherein said biological sample is selected from the group consisting of plasma, PBMCs, whole blood, serum and peripheral blood, or a combination thereof.

10. An in vitro method according to anyone of claims 5-9, wherein the detection of the binding of said one or more antigens to the antibodies is performed by ELISA or Western blotting.

11. An in vitro method according to anyone of the claims 5-10, wherein said cancer overexpressing sodium channels is selected from the group consisting of breast cancer, colon cancer, prostate cancer, cervical cancer, melanoma, mesothelioma, neuroblastoma, non-small cell lung cancer, ovarian cancer, thyroid cancer, small-cell lung cancer and lymphoma.

12. A diagnostic kit comprising one or more antigens that specifically binds to the antibodies against NaV 1.5 channel (SEQ ID NO:1) and/or related isoforms according to anyone of claims 1-4, for the prediction of the risk of cardiac arrythmia or sudden cardiac death in a patient suffering from cancer overexpressing sodium channels.

13. The diagnostic kit for use according to claim 12, wherein said one or more antigens are antigenic fragments belonging to a binding site of the extracellular loops of the Nav 1.5 channel, said binding site of the extracellular loops being selected from the group of sequences SEQ ID NO:2-SEQ ID NO:7.

14. The diagnostic kit for use according to anyone of claims 12-13, wherein said antigens are labelled or attached to a solid support.

15. Use of one or more of the antibodies against NaV 1.5 channel (SEQ ID NO:1) and/or related isoforms according to anyone of the claims 1-4 for the prediction of risk of cardiac arrythmia or sudden cardiac death in a patient suffering from cancer overexpressing sodium channels, wherein the detection of the presence of such antibodies in a biological sample of said patient indicates an elevated risk, wherein said biological sample is selected from the group consisting of plasma, PBMCs, whole blood, serum and peripheral blood, or a combination thereof.

16. Use according to claim 15, wherein said detection is carried out by using one or more antigens that specifically binds to the autoantibodies against NaV 1.5 channel (SEQ ID NO:1) and/or related isoforms.

17. Use according to claim 16, wherein said one or more antigens are antigenic fragments belonging to a binding site of the extracellular loop of NaV 1.5 channel and/or related isoforms selected from the group comprising the following the sequences SEQ ID NO:2-SEQ ID NO:7.
